# EUROPEAN PATENT SPECIFICATION

(11) **EP 1 235 905 B1**
(45) Date of publication and mention of the grant of the patent: **25.02.2009**
(21) Application number: 00986128.7
(22) Date of filing: 06.12.2000
(51) Int. Cl.: C12N 15/00, G01N 1/40, G01N 1/34

(54) **DEVICE FOR EXTRACTION OF NUCLEIC ACIDS**
VORRICHTUNG ZUR EXTRAKTION VON NUKLEINSäUREN
APPAREIL POUR L'EXTRACTION D'ACIDES NUCLEIQUES

(30) Priority: 10.12.1999 SE 9904539
(43) Date of publication of application: 04.09.2002
(73) Proprietor: ALPHAHELIX MOLECULAR DIAGNOSTICS AB, 751 83 Uppsala (SE)
(72) Inventor: MALMQUIST, Mats, S-754 39 Uppsala (SE)
(74) Representative: Carlsson, Carl Fredrik Munk
(86) International application number: PCT/SE2000/002448
(87) International publication number: WO 2001/042487

(56) References cited:
- EP-A2- 0 251 689
- US-A- 4 722 792
- US-A- 5 330 916
- US-A- 5 647 990
- US-A- 5 728 267
- US-A- 5 786 182

## Description

### Field of the invention

The present invention concerns closed or so called dry handling of reagents and samples, and in particular the field of sample preparation, quantitative and qualitative extraction, purification and amplification of nucleic acids (DNA or RNA, including different species of nucleic acids) from organic samples, such as blood, serum, urine, cell suspensions, *in vitro* amplified samples and biopsy samples.

### Background of the invention

There are a large number of different protocols for the isolation and purification of nucleic acids. Most of the methods aim at the isolation of highly purified samples, suitable for use in PCR amplifications.

One presently used protocol, utilising the Split Second^{™} DNA Preparation Kit (Boehringer Mannheim GmbH) comprises the following steps: a first buffer solution is dispensed in microcentrifuge tubes. The sample, e.g. human whole blood, is added to the tubes. The tubes are placed on a rocking platform for 10 minutes and then centrifuged at 2500 rpm for 5 minutes in a microfuge. The supernatant is then removed and discarded, and the pellet resuspended in a buffer solution. The resuspended pellet is then centrifuged at 2500 rpm for 3 minutes. Following this second centrifugation, the supernatant is removed and discarded. The pellet is resuspended in a second buffer and the mixture vortexed thoroughly. After incubation for 5 minutes in a 65°C water bath, the sample can be used directly for PCR.

Another protocol is the purification of DNA using a chaotropic agent and silica particles, as described by Dr. J. Kleiber (Preparation of DNA templates, Boehringer Mannheim GmbH, FRG). Silica suspension is added to lysis buffer in a microcentrifuge tube, and vortexed. EDTA-treated blood is then added to the lysis buffer containing the silica and the mixture vortexed. After a 10 minute incubation, during which the tube should be inverted regularly to prevent the silica from settling, the mixture is centrifuged. After removing the supernatant, the silica-nucleic acid pellet is subjected to a series of washes: first a wash buffer (guanidine thiocyanate, Tris/HCl), then ethanol (70 %) and finally acetone. Each wash step requires vortexing of the mixture, centrifugation and removal of the supernatant. After the last wash, the silica-nucleic acid pellet is dried by heating it at 56°C for 10 min. Then, the pellet is resuspended in TE-buffer and the mixture incubated for 10 min at 56 °C. Finally, the mixture is centrifuged at 10.000 x g for 2 minutes. The supernatant will now contain the purified nucleic acid and it can be used for PCR.

It is easily recognised, that the multiple steps of the presently used protocols are labor intensive and require relentless concentration. Exact volumes have to be pipetted in a large number of microcentrifuge tubes and these tubes are subjected to different steps, such as centrifugation, vortexing and incubation.

The handling of samples taken from the human body, regardless if they are biopsy samples, blood or serum samples, or samples of other body fluids such as urine or saliva, cell suspensions and *in vitro* amplified samples, involves many practical complications and special considerations. Not only must the laboratory personnel be protected from disease causing agents, possibly contained in the samples, the samples themselves must be protected from contamination, either from other samples or from the personnel handling the samples.

The many steps involved in conventional nucleic acid purification increase the risk of transmission of nucleic acids from sample to sample. In determinations involving the extremely sensitive polymerase chain reaction (PCR) or other amplification systems, the slightest contamination can result in false-positive results.

The handling of human blood poses specific problems. As heparinised blood cannot be used (heparin inhibits the PCR) the samples tend to coagulate in the containers containing the samples, in pipette tips and microcentrifuge tubes. Further, blood has a tendency to adhere to surfaces and to dry, forming minute flakes, which easily become airborne. Finally, the psychological discomfort or stress, experienced when handling potentially contagious human blood samples, should not be neglected.

The present invention aims at improving, e.g. simplifying the purification of nucleic acids from organic samples in general and in particular the preparation of nucleic acid samples from human whole blood.

Notably, nucleic acids are present in the form of deoxyribonucleic acid (DNA) and ribonucleic acid (RNA). DNA can in turn be subdivided into plasmide DNA and genomic DNA. RNA is usually subdivided into messenger RNA (RNA), transfer RNA (tRNA) and ribosomal RNA (rRNA). These groups are frequently referred to as "nucleic acid species". It would be desirable to make available an easy and reliable method for the qualitative extraction of nucleic acids, i.e. the differentiation between different nucleic acid species.

In many chemical and pharmaceutical applications, a reagent or pharmaceutical agent is stored and delivered to the user in lyophilised form and has to be reconstituted by mixing it with a suitable amount of an appropriate solvent. The solvent is often water or an aqueous solution, such as physiological saline solution. This technique is resorted to mainly in order to extend the stability and thus the storage life of the reagent or pharmaceutical agent. In some cases, a reaction between components, contained in the reagents, is made impossible or considerably retarded in a lyophilised state and initiated when the material is reconstituted.

When reconstituting a lyophilised substance, thorough mixing must be guaranteed. Concentration gradients, insufficient mixing and perhaps remaining solids can cause errors and risks in the further use of the reconstituted solution. The risk of contaminating the lyophilised substance must be taken into account, in particular when the reconstitution is done openly.

Examples of applications where contaminating material can totally disarrange the result include analytical and biochemical applications where genetic information from a sample is amplified. Important reactions of this type include the polymerase chain reaction (PCR), ligase chain reaction (LCR), "gapped-LCR-reaction", nucleic acid sequence-based amplification (NASBA), self-sustained replication (SSR), transcription mediated amplification (TMA), strand displacement amplification (SDA), target amplification, signal amplification, Hybrid Capture^{®} reaction or a combination of any of the above. The present invention aims at minimizing or totally avoiding contamination in the preparation of samples for the above applications and others, where contamination causes risks for errors.

Another aim of the present invention is thus to make available a method and device for restitution of lyophilised substances, e.g. reagents for use in biochemical analyses or pharmaceutical agents, offering a higher degree of safety, reliability and user-friendliness than presently available systems.

### Closest prior art

U.S. 5.330,916 discloses a method for the extraction of cellular components and a vessel suitable for use in this method. The so called double-ended extraction vessel has two compartments, separated by a filter, and a grinder - plunger movably positioned in one of the chambers. By moving the grinder - plunger, the sample is subjected to mechanical forces, breaking open the cells in the sample, releasing the cell content into an aqueous phase. Said aqueous phase passes the filter into the second compartment, the organic phase and cellular debris remaining in the first compartment.

U.S. 5,786,182 discloses a dual chamber disposable vessel for amplification reactions, wherein a first chamber contains an amplification reagent mix and a second chamber contains an amplification enzyme. These two chambers are connected by a fluid channel which can be opened or through which the sample can be forced to pass as the result of mechanical action, application of vacuum etc. The gist of the invention is to separate reagents with different resistance to heat, i.a. a heat labile enzymatic reagent and a heat stable amplification reagent, and to make it possible to bring them together at a chosen moment.

The vessel according to U.S. 5,786,182 is directed to amplification reactions and does not include the possibilities of interchanging the chambers constituting the dual chamber vessel, nor is it suitable for the extraction of nucleic acids, i.a. as it lacks means for a thorough mixing of the sample and reagent.

The method and vessel according to 5,330,916 constitutes the closest prior art as it concerns the extraction of nucleic acids. It however does not make it possible to customise the extraction procedure, exchange of buffers, sequential extraction etc in an easy and reliable manner.

### Summary of the invention

The present invention solves the above problems by making available devices and methods according to the attached claims.

The present invention makes available a device for the extraction of nucleic acids, wherein said system comprises a first vessel (3) for containing the sample and at least one second vessel (4), containing at least one reagent (2), said vessels being detachably connected via a narrow passage (7. 8), and means (5, 6) for forcing the sample from the first vessel into the second vessel and back.

The present invention also makes available a method for the extraction of nucleic acids from a sample, wherein said sample is provided in a first vessel, which is detachably connected to a second vessel, said second vessel containing a predispensed reagent, whereupon the sample is pumped into the second vessel and back, repeating this until sufficiently mixed, whereupon the vessel containing the mixture is attached to a third vessel, containing a predispensed reagent and the mixing repeated.

Further embodiments and their advantages of the invention will be evident or deducible from the description and examples, including the attached figures.

### Short description of the drawing

The present invention will be disclosed in closer detail in the description and examples below, with reference to the accompanying drawing, in which
**Figure 1** shows a cross section of two vessels, A and B, according to one embodiment of the invention;
**Figure 2** shows the two vessels of fig. 1 interconnected according to the invention;
**Figure 3** shows a vessel with a cap for separation of the matrix, carrying the nucleic acids; and
**Figure 4** shows an assembly of fig. 3, placed in a centrifuge tube (A) and a microcentrifuge tube (B), respectively, for example for centrifugal emptying.

### Description

The inventive method for purification, extraction and enzymatic treatment of nucleic acids according to the present invention makes possible the "dry" handling of a sample and necessary buffers, as pipetting steps become unnecessary.

A sample, e.g. a volume of blood, serum, urine, saliva, a cell suspension, e.g. from a biopsy sample, or a sample amplified *in vitro,* is placed in a first space. This first space can be a space contained in a test tube, a Vacutainer^{®}, a syringe or preferably a first space, contained in a device according to the inven ion and described below. The sample is then brought in contact with a lysis buffer, which preferably also contains a matrix, capable of binding nucleic acids. A suitable lysis buffer is a salt sclution, further containing a detergent, conventionally used agents, such as Tris (Boehringer Mannheim GmbH) and EDTA (Merck). A suitable matrix consists of glass or silica particles, diatoms, glass fibres, nylon fibres, cellulose slurry, para-magnetic beads, latex beads etc. The matrix can also be coated, e.g. coated with streptavidin or any other coating material for separating nucleic acid strands or a single nucleic acid strand exclusively.

This first buffer, preferably also comprising a solid matrix, is contained in a second space, having a narrow passage. When entering the second space, the sample forms a mixture with the lysis buffer and the matrix. This mixture is then forced to pass the above mentioned narrow passage and enters a space, which can be the first space, in case the sample was originally provided in a device according to the invention. When passing the narrow passage, or opening between the two spaces or vessels, the mixture is efficiently mixed. The mixture is then forced back into the second space and the procedure is repeated a number of times, guaranteeing a thorough mixing and lysis of the cells in the sample. After a sufficient number of passes, the mixture is practically homogenous. During this mixing, the nucleic acids bind to the matrix, contained in the lysis buffer. The mixing is terminated and the mixture contained in either the first or the second space.

The matrix with bound nucleic acids is then separated from the bulk of solution. In the case of using a particulate matrix, this separation can be achieved by closing the space containing the matrix with a membrane or with a separating cap, as described below, and centrifuging the device. The matrix remains on the membrane or in the separating cap.

When using a para-magnetic matrix, separation is achieved by applying a magnetic field around the vessel or in the movable element or plunger, used to force the contents of the vessels to pass from one vessel to another.

The mixture containing the lysis buffer and the matrix is then mixed with a rinse buffer, contained in a third space or vessel, connected to the second space or vessel. By forcing the mixture to pass back and forth between these two spaces, through a narrow passage, thorough mixing and rinsing is again ensured. The mixing is terminated and the mixture contained in either the second or third space, the other being discarded.

Finally, the purified nucleic acids can be removed from the matrix using an eluation buffer or in case of a para-magnetic matrix, by removing the magnetic field.

By using different matrixes, the extraction of different nucleic acid species can be achieved. This is done by choosing the material of the matrix, the structure of the matrix, its packing and other structural / physical properties. It can also be influenced by selecting appropriate chemical properties and/or chemical or biological pre-treatment, such as affinity favouring treatments, coating the matrix with antibodies, affibodies, streptavidin, biotin. Specific nucleic acid sequences can be extracted using complementary nucleic acid hybridised to the matrix. This way for example specific viral nucleic acids can be extracted as part of the preparation of the sample for a diagnostic test.

The extraction buffer or buffers can also be chosen or adapted to the extraction of specific nucleic acid species. According to one embodiment of the invention, a sample is passed back and forth between two vessels, one containing a matrix specifically adapted to or favouring the adsorption-of one nucleic acid species, the other vessel containing a matrix specifically adapted to or favouring the adsorption of another nucleic acid species, and a buffer suitable for both matrixes. This way the extracted nucleic acid species are physically separated, which improves the kinetics of the reaction and leads to higher yields, both quantitatively and qualitatively.

According to an alternative embodiment of this method, the matrix can be added after lysis has been performed.

The device according to the present invention comprises at least two vessels having movable wall portions or elements for expelling the content of each vessel, and couplings that fit the corresponding part of at least the other vessel and preferably further vessels.

According to a preferred embodiment, one vessel contains a solid matrix permanently fixed to the vessel or physically hindered from leaving the vessel. This matrix can be a plug of a particulate or fibrous material, e.g. glass or silica particles, glass fibre, nylon fibre, cellulose or diatoms, optionally sintered or otherwise compressed to form a plug-like shape, unable to leave the vessel in the direction of the fluid stream when the vessel is emptied. The matrix can be specifically chosen, adapted or modified, with consideration to its physical / structural and/or chemical / biological properties, as described above.

Alternatively, when using para-magnetic particles, the movements of the matrix particles is regulated by applying a magnetic field to the vessel.

The matrix may also constitute a coating on the inside walls of one of the vessels, alternatively the walls of one of the vessels are made of glass, acrylic, polystyrene or other material known to bind reversibly e.g. to nucleic acids under specific conditions. In the latter case, some surface treatment, such as rugging or other enhancement of the active area of the walls, is preferred.

According to one embodiment of the invention, the possibilities of combining the vessels is restricted by the thread, shape or size of their nozzles or collars. A first vessel can for example be equipped with both an outside and an inside thread.-A second vessel to be connected to said first vessel engages the inner thread. After the mixing involving the first vessel is terminated, the second vessel is discarded and the first vessel connected to a third vessel, engaging its outer thread.

Further, using colour codes, tactile marks and the like, the order of using the different vessels can be guided. By posing direct physical restrictions (different threads, different gauges of parts to be connected etc) on the coupling of the vessels, the possibility of errors is minimised.

The device according to the present invention is preferably made of a suitable thermoplastic. Examples of such materials include, but are not limited to, polypropylene (PP), polystyrene (PS), polyethylene (PE), high density polyethylene (HDPE), polycarbonate (PC), polyacetate (PA), poly-methylene-methacrylate (PMMA) and polyvinylidene-fluoride (PVDF). The choice of material is not only governed by chemical and thermal requirements, due to the reagents and buffers to be handled or the reactions to be performed in the device, but also economic considerations, such as material costs, production technology etc. One suitable method of production is injection moulding. Vacuum die-casting is another possible method of production. The inventive device is of course manufactured under conditions rendering it free from contaminants, which possibly influence the reaction or reactions it is intended to be used in.

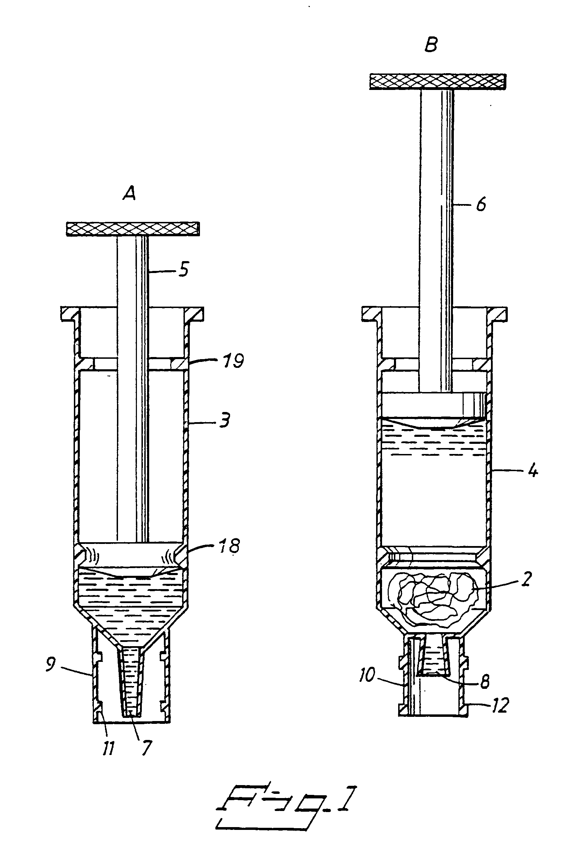

The device is shown in Fig. 1 as two vessels 3 and 4. For the purpose of illustration, the vessels are shown as artefacts resembling conventional syringes, having a body, a movable plunger 5 and 6, an exit opening or nozzle 7 and 8. Around the exit openings, a collar 9 or 10 is arranged. As the collar on vessel 3 has inner threads 11, and the collar on vessel 4 has corresponding outer threads 12, they can be securely connected to each other, ensuring a tight fit between the openings 7 and 8. The collars and threads can also be used for attaching a cover or cap to the vessels, protecting the integrity of their content, ensuring the sterility of the inner surfaces etc.

For illustrative purposes, the liquid contained in 3, in the volume indicated by dashed lines in the figure, can be held to be a sample, for example a whole blood sample. The liquid in 4, is then a lysis buffer, preferably also containing a matrix 2.

In order to regulate the movement of the plungers, the vessels here have a first annular edge 18 in the proximal end of the vessel and a second annular edge 19 in the distal end of the vessel. The first edge 18 will give a noticeable resistance and thus indicate to the user, that the plunger is near its lowest position. It is however possible to press the plunger past the first edge 18, for example when emptying the vessel. The second edge 19 will prevent the plunger from being withdrawn from the vessel, for example by mistake or as a result of too high pressure building up in the vessel.

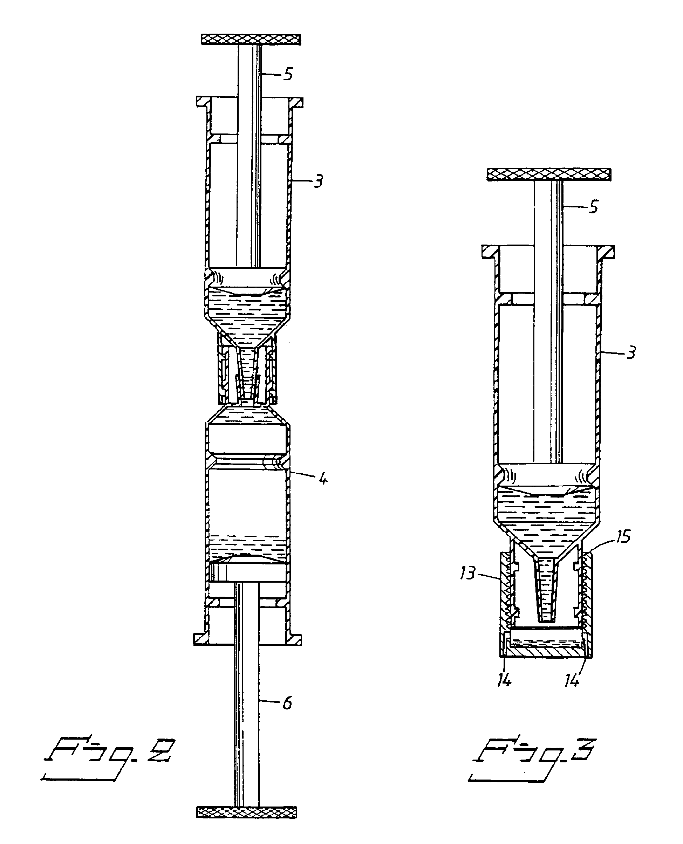

In Fig. 2, two vessels 3 and 4 are shown when connected using the threaded collars described above. The connection opens a fluid path between the space of the two vessels, passing a narrow constriction or "neck" formed by the exit openings of the vessels. As the connection is tight, the two vessels define one volume. When the plunger 5 is depressed, the sample contained in vessel 3 is forced through the narrow passage into vessel 4, which contains the lysis buffer and matrix 2. As the connection is tight, the plunger 6 is forced back, by the pressure exerted by plunger 5. When most of the sample is emptied into vessel 4, the plunger 6 is depressed, forcing sample plus buffer into vessel 3. This way the contents are pumped back and forth between vessels 3 and 4, forming a mixture. When using non-heparinised blood - as necessary for PCR purposes - this mixing has shown to efficiently prevent the blood from coagulating. The mixing is terminated leaving the mixture in one of the vessels, either 3 or 4.

In an embodiment where vessel 4 contains a matrix unable to leave the vessel, e.g. a plug of glass fibres 2 or sintered silica particles etc, it is preferred that, after thorough mixing, the contents of 4 are emptied into 3, or another equivalent vessel, leaving only the matrix with bound nucleic acids. Vessel 3 is then discarded, together with its contents.

A third vessel (not shown) containing a rinse buffer is then attached to vessel 4 and the pumping procedure repeated. When the rinse is terminated, vessel 4 is emptied of all but the matrix with the bound nucleic acids and the third vessel, containing the used rinse buffer is discarded.

The nucleic acids can then be eluated from the matrix, for example by filling vessel 4 with an eluation buffer, preferably contained in a fourth vessel, according to the invention. Alternatively, a vessel containing an eluation buffer is connected to vessel 4 and the eluation performed by pumping the contents back and forth between these two vessels.

If the extracted nucleic acid is to be used as a template for an amplification process, e.g. PCR, a fourth vessel containing lyophilised reagents for the amplification can be connected to vessel 4. The content of vessel 4, including the eluated DNA is then forced into the fourth vessel and pumped back and forth, thus effectively solving and homogenising the reagents. In this embodiment, a complete reaction mixture including template for the amplification is prepared and dispensed directly to the reaction vessel used for PCR. This offers many benefits, as simplified handling and reduced risk of contamination etc.

The vessels can also be emptied by centrifugation. Fig. 3 shows an embodiment with a vessel 3 containing a mixture of buffer and matrix. At this stage, the nucleic acids are bound to the matrix and an eluation buffer added. A separation cap 13 having exit pores 14 is attached to the exit opening 7 of the vessel 3. The cap 13 can be secured to threads on the collar 9 (not shown). When centrifuged, the eluation buffer leaves the vessel through the exit pores 14, leaving the matrix in the separation cap. The eluated nucleic acids can then be used for amplification, e.g. PCR.

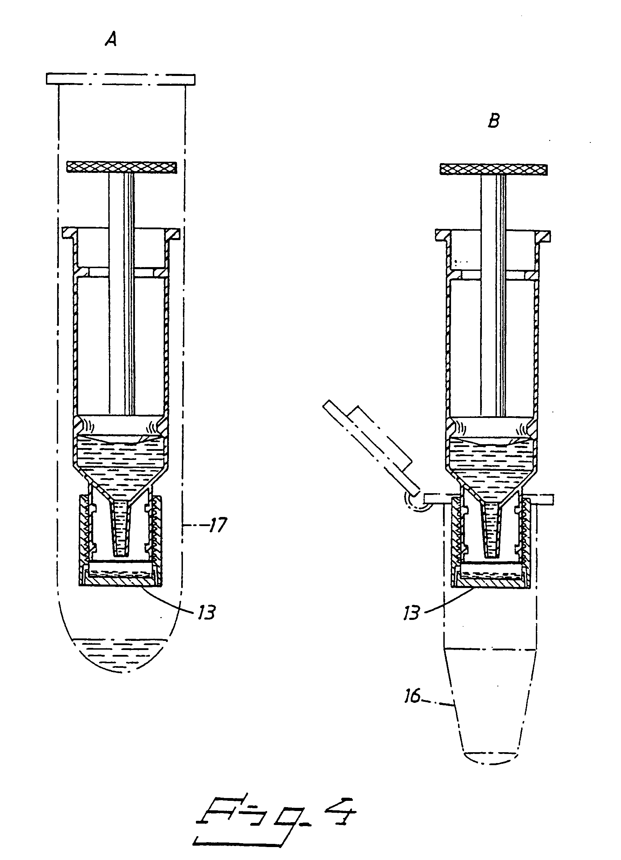

Fig. 4 shows two applications, one where a vessel with a separation cap 13 attached, is arranged in a centrifuge tube 17, and one where the vessel is arranged to empty its contents into a microcentrifuge tube 16. During centrifugation, the matrix remains in the vessel or is held back by the separation cap, while the eluation buffer and the nucleic acids pass the exit pores 14 and are transferred to the tube 16 or 17. The tubes 16 and 17 can then be subjected to further steps, e.g. as required by the PCR protocol.

The first vessel, which is the container of the sample, can also be the primary receptacle of the sample. It is conceived, that the first vessel is adapted for sample collection. According to one embodiment of the invention, the first vessel is adapted for holding a hypodermic needle. This way a blood sample can be taken directly in the first vessel. According to another embodiment of the invention, the first vessel is adapted for taking a band from a gel or a colony from an agar plate. The vessel is then equipped with a nozzle for punching a band or a nozzle for lifting a colony from an agar plate. The pumping of the contents between vessels, as in the method described above, is very suitable for extracting nucleic acids from a gel or a clone grown on an agar plate.

According to a further embodiment, the inventive device and method can be adapted to a parallel format. In laboratories handling large numbers of samples, an embodiment having a manifold of vessels, e.g. 96, 384 or more vessels of microcentrifuge type or microtitre wells, can be assembled into the so called microtitre standard format or other parallel formats. Such an assembly of e.g. 8 x 12 vessels is preferrably injection moulded in one single piece. Corresponding plungers are also assembeld to form rows and columns in the same format, and also preferrably injection moulded in one single piece. The pumping described above can be performed manually. Preferrably, an automatic device is used. This can be a mechanic or pneumatic device, engaging the assembly of plungers. In this embodiment, the exit openings 7, 8 and the collars 9, 10 having threads, should be modified in a way permitting secure and tight connection without twisting. A twisting movement is difficult to perform when the vessels are assembled in a grid as in this embodiment. In other words, the vessels should be tightly engaged by aligning the vessels and pressing them together. This can be achieved using tight fitting connections, flanges, "snap-lock" mechanisms etc. A "push-and-lock" and "pull-and-release" mechanism can be devised by changing the shape of the collars 9 and 10.

### Example

In the present example, a set of interconnectable syringes were used. Different matrixes were tested: silica particles, glass and nylon fibres. A lysis buffer consisting of a salt solution and detergent was used. The rinse buffer consisted of a salt solution and ethanol.

A sample of non-heparinised human whole blood was drawn into a first syringe, either directly from a patient, using a hypodermic needle, attached to the syringe, or from an intermediate container, a Vacutainer^{®}, containing a patient sample.

A second syringe was provided, containing silica particles suspended in a lysis buffer. The syringe containing the blood sample was then connected to the second syringe, and the blood sample emptied into the lysis buffer. The mixed content of the two syringes was then pumped back and forth between the two syringes. Each passage through the narrow waist of the interconnected syringes helped to mix the sample with the lysis buffer and ensured thorough mixing of the silica particles in the solution. This pumping was repeated for a few minutes, and the mixing terminated with the sample and lysis buffer being entirely in one of the syringes. The other syringe was detached and discarded.

Using a mixture of detergent and salt solution for the lysis of cells in a sample of non-heparinised blood, it was found that the coagulation of the blood was effectively prevented by the inventive treatment.

A third syringe, containing a rinse buffer was connected to the syringe containing the sample, silica and lysis buffer. The rinse buffer was then pumped back and forth between the third and second syringe. After the mixing was terminated, the combined solution was left in the second syringe.

When using a particulate matrix, moving freely in the contents of the vessel, the matrix was separated from the solution by attaching a separation cap on the tip of the syringe, placing the same in a centrifuge tube and subjecting it to centrifugation (as schematically shown in the attached fig. 4 of the drawings). The matrix remained in the separation cap whereas the solution was removed. A further rinse can be performed or the nucleic acid eluated from the pellet, present in the separation cap.

When the matrix was a glass fibre plug - or, as in a second example performed by the inventor, a length of nylon thread - no separation cap needs to be used. Instead, the matrix was rinsed and eluated *in situ,* by attaching different syringes containing the necessary buffers, to the syringe containing the matrix. Finally, the nucleic acids were eluated from the matrix. The purification result was confirmed by subjecting the eluated sample to gel electrophoresis. A distinct band showed that the purification of nucleic acids had been successful.

Although the invention has been described with regard to its preferred embodiments, which constitute the best mode presently known to the inventor, it should be understood that various changes and modifications as would be obvious to one having the ordinary skill in this art may be made without departing from the scope of the invention as set forth in the claims appended hereto.

## Claims

1. A device for the extraction of nucleic acids in organic samples, **characterized in that** said device comprises a first vessel (3) for containing the sample, a first plunger (5) for expelling said sample from said first vessel, and at least one second vessel (4), containing at least one predispensed reagent (2), and having a second plunger (6) for expelling said reagent from said second vessel, said first and said at least one second vessel being detachably connected via a narrow passage (7, 8), and said first and said at least one second vessel defining one closed volume.

2. Device according to claim 1, **characterized in that** the predispensed reagent in said second vessel is a lyophilised reagent.

3. Device according to claim 1, **characterized in that** said second vessel further contains a matrix with nucleic acid binding properties.

4. Device according to claim 1, **characterized in that** the predispensed reagent in said second vessel is a lysis buffer and that said second vessel further contains a matrix with nucleic acid binding properties.

5. Device according to any one of claim 3 or 4, **characterized in that** said matrix is a particulate matrix moving freely in the vessel.

6. Device according to any one of claim 3 or 4, **characterized in that** said matrix consists of primary particles or fibres, forming a secondary shape which is prevented from leaving the vessel.

7. Device according to claim 5, **characterized in that** said matrix is chosen among glass or silica particles, diatoms, glass fibres, nylon fibres, cellulose slurry, para-magnetic beads and latex beads.

8. Device according to claim 3, **characterized in that** said matrix is given a specific affinity favouring treatment.

9. Device according to claim 3, **characterized in that** said matrix is prevented from leaving the vessel by a magnetic field acting on magnetic components of the matrix.

10. Device according to claim 3, **characterized in that** the matrix is a plug of glass fibres.

11. Device according to claim 8, **characterized in that** the matrix is coated with streptavidin.

12. Device according to claim 8, **characterized in that** the matrix carries hybridised nucleic acids.

13. Device according to claim 1, **characterized in that** the first vessel (3) has connecting means (9, 11) for engaging corresponding means (10, 12) on a second (4) vessel or further vessels.

14. Device according to claim 13, **characterized in that** said connecting means (9, 11, 10, 12), by their size and/or function, regulate the sequence of attachment of the second and further vessels.

15. Device according to claim 1, **characterized in that** the vessels (3, 4) and further vessels attachable to these, have physical properties such as tactile marks, colour codes and the like, guiding their sequential use.

16. Device according to claim 3, **characterized in that** it comprises a detachable separation cap (13) which retains the solid matrix.

17. Kit comprising a device according to claim 1 and a device (13) for separation of a nucleic acid binding matrix from a liquid, said device having an open end for attaching to a device according to claim 1, and a closed end provided with at least one exit pore, wherein each of said at least one exit pore (s) is (are) situated above the lowest inner surface of the device, defining a volume which is not emptied by centrifugation.

18. Kit according to claim 17, **characterized in that** it attaches securely to the end (9 or 10) of a device (3 or 4) according to claim 1 and fits into a centrifuge tube.

19. Device according to any one of claim 1-16, **characterized in that** the vessels are connected to form a grid, corresponding to the microtitre standard format.

20. Kit according to any one of claim 17-18, **characterized in that** the vessels are connected to form a grid, corresponding to the microtitre standard format.

21. Method for nucleic acid extraction from an organic sample, **characterized in that** a device according to any one of claims 1 - 16 is used.

22. Method for nucleic acid extraction from an organic sample, **characterized in that** a kit according to any one of claims 17 - 18 is used.

23. Method for nucleic acid extraction from an *in vitro* amplified sample, **characterized in that** a device according to any one of claims 1 - 16 is used.

24. Method for nucleic acid extraction from an *in vitro* amplified sample, **characterized in that** a kit according to any one of claims 17 -18 is used.

25. Method according to claim 21 or 22, **characterized in that** said sample is one of blood, serum, urine, saliva, a cell suspension and a biopsy sample.

26. Method according to claim 21 or 22, **characterized in that** said sample is human whole blood.

27. Method for reconstitution of a lyophilised reagent, **characterized in that** a device according to claim 1 is used.

28. Method for the reconstitution of a reagent for any one of the following reactions: polymerase chain reaction (PCR), ligase chain reaction (LCR), "gapped-LCR-reaction", nucleic acid sequence-based amplification (NASBA), self-sustained replication (SSR), transcription mediated amplification (TMA), strand displacement amplification (SDA), Hybrid Capture^{®} reaction, target amplification, signal amplification, or a combination thereof, **characterized in that** a device according to claim 1 is used.

29. A method for the extraction of nucleic acids from a sample, **characterized in that** said sample is provided in a first vessel, which is detachably connected to a second vessel, said second vessel containing a predispensed reagent, whereupon the sample is pumped into the second vessel and back, repeating this until sufficiently mixed, whereupon the vessel containing the mixture is attached to a third vessel, containing a predispensed reagent and the mixing repeated.

30. Method according to claim 29, **characterized in that** the second vessel contains a lysis buffer and a matrix, capable of binding nucleic acids.

31. Method according to claim 29, **characterized in that** the third vessel contains a rinse buffer.

32. Method according to any one of claims 29 - 31, **characterized in that** the purified sample is removed from a vessel (3, 4) by centrifugation.

33. Method according to any one of claims 29- 31, **characterized in that** the matrix is separated from the liquid it is suspended in by centrifugation using a device according to claim 17.

34. Method according to any one of claims 29-31, **characterized in that** the matrix is separated from the liquid it is suspended in by use of a magnetic field.

35. Method according to any one of claims 29-31, **characterized in that** the sample is one of the following: a sample of blood, serum, urine, saliva, a cell suspension, an *in vitro* amplified sample or a biopsy sample.

36. Method according to any one of claims 29 - 31, **characterized in that** the sample is a human whole blood sample.

37. Method for the extraction of specific nucleic acid species, **characterized in that** a device according to claim 1 is used, said device containing a matrix treated in a manner favouring the attachment of said nucleic acid species to said matrix.

38. Method for the extraction of specific nucleic acid sequences, **characterized in that** a device according to claim 1 is used, said device containing a matrix having a complementary nucleic acid sequence hybridised thereon.

## Patentansprüche

1. Vorrichtung zur Extraktion von Nukleinsäuren in organischen Proben, **dadurch gekennzeichnet, dass** die Vorrichtung ein erstes Gefäß (3), um die Probe zu enthalten, einen ersten Kolben (5), um die Probe aus dem Gefäß auszustoßen, und mindestens ein zweites Gefäß (4) umfasst, das mindestens ein im Voraus dispensiertes Reagens (2) enthält , und einen zweiten Kolben (6) umfasst, um das Reagens aus dem zweiten Gefäß auszustoßen, wobei das erste und das mindestens eine zweite Gefäß über einen engen Übergang (7, 8) abnehmbar verbunden sind und das erste und das mindestens ein zweite Gefäß ein geschlossenes Volumen definieren.

2. Vorrichtung nach Anspruch 1, **dadurch gekennzeichnet, dass** das im Voraus dispensierte Reagens im zweiten Gefäß ein gefriergetrocknetes Reagens ist.

3. Vorrichtung nach Anspruch 1, **dadurch gekennzeichnet, dass** das zweite Gefäß weiter eine Matrix mit Bindeeigenschaften für Nukleinsäure enthält.

4. Vorrichtung nach Anspruch 1, **dadurch gekennzeichnet, dass** das im Voraus dispensierte Reagens im zweiten Gefäß ein Lysepuffer ist, und dass das zweite Gefäß weiter eine Matrix mit Bindeeigenschaften für Nukleinsäure enthält.

5. Vorrichtung nach einem der Ansprüche 3 oder 4, **dadurch gekennzeichnet, dass** die Matrix eine partikuläre Matrix ist, die sich im Gefäß frei bewegt.

6. Vorrichtung nach einem der Ansprüche 3 oder 4, **dadurch gekennzeichnet, dass** die Matrix aus primären Partikeln oder Fasern besteht, die eine sekundäre Form bilden, die daran gehindert wird, das Gefäß zu verlassen.

7. Vorrichtung nach Anspruch 5, **dadurch gekennzeichnet**, das die Matrix aus Glas- oder Siliziumpartikeln, Diatomen, Glasfasern, Nylonfasern, Zellulosebrei, paramagnetischen Kügelchen und Latexkügelchen gewählt wird.

8. Vorrichtung nach Anspruch 3, **dadurch gekennzeichnet, dass** die Matrix einer spezifischen, die Affinität begünstigenden Behandlung unterzogen wird.

9. Vorrichtung nach Anspruch 3, **dadurch gekennzeichnet, dass** die Matrix durch ein magnetisches Feld, das auf magnetische Bestandteile der Matri x wirkt, daran gehindert wird, das Gefäß zu verlassen.

10. Vorrichtung nach Anspruch 3, **dadurch gekennzeichnet, dass** die Matrix ein Pfropfen aus Glasfasern ist.

11. Vorrichtung nach Anspruch 8, **dadurch gekennzeichnet, dass** die Matrix mit Streptavidin beschichtet ist.

12. Vorrichtung nach Anspruch 8, **dadurch gekennzeichnet, dass** die Matrix hybridisierte Nukleinsäuren trägt.

13. Vorrichtung nach Anspruch 1, **dadurch gekennzeichnet, dass** das erste Gefäß (3) Verbindungsmittel (9, 11) aufweist, um in entsprechende Mittel (10, 12) auf einem zweiten (4) Gefäß oder weiteren Gefäßen einzugreifen.

14. Vorrichtung nach Anspruch 13, **dadurch gekennzeichnet, dass** die Verbindungsmittel (9, 11, 10, 12) durch ihre Größe und/oder Funktion die Anheftungssequenz des zweiten und weiterer Gefäße regulieren.

15. Vorrichtung nach Anspruch 1, **dadurch gekennzeichnet, dass** die Gefäße (3, 4) und weitere Gefäße, die an diese angebracht werden können, physische Eigenschaften aufweisen, wie z.B. taktile Markierungen, Farbcodes und Ähnliches, die ihre sequenzielle Verwendung leiten.

16. Vorrichtung nach Anspruch 3, **dadurch gekennzeichnet, dass** sie eine abnehmbare Trennklappe (13) umfasst, die die feste Matrix zurückhält.

17. Kit, umfassend eine Vorrichtung nach Anspruch 1 und eine Vorrichtung (13) zum Trennen der Bindematrix einer Nukleinsäure von einer Flüssigkeit, wobei die Vorrichtung ein offenes Ende zum Anbringen an eine Vorrichtung nach Anspruch 1 umfasst, und ein geschlossenes Ende, ausgestattet mit mindestens einer Ausgangspore, wobei sich jede der mindestens einen Ausgangspore über der niedrigsten Innenfläche der Vorrichtung befindet, wodurch ein Volumen definiert wird, das nicht durch Zentrifugierung geleert wird.

18. Kit nach Anspruch 17, **dadurch gekennzeichnet, dass** es sicher an das Ende (9 oder 10) einer Vorrichtung (3 oder 4) nach Anspruch 1 anschließt und in ein Zentrifugenrohr passt.

19. Vorrichtung nach einem der Ansprüche 1 - 16, **dadurch gekennzeichnet, dass** die Gefäße verbunden sind, um ein Gitter zu bilden, entsprechend dem Mikrotiter-Standardformat.

20. Kit nach einem der Ansprüche 17 - 18, **dadurch gekennzeichnet, dass** die Gefäße verbunden sind, um ein Gitter zu bilden, entsprechend dem Mikrotiter-Standardformat.

21. Verfahren zur Extraktion von Nukleinsäure von einer organischen Probe, **dadurch gekennzeichnet, dass** eine Vorrichtung nach einem der Ansprüche 1-16 verwendet wird.

22. Verfahren zur Extraktion von Nukleinsäure von einer organischen Probe, **dadurch gekennzeichnet, dass** ein Kit nach einem der Ansprüche 17-18 verwendet wird.

23. Verfahren zur Extraktion von Nukleinsäure von einer *in vitro* amplifizierten Probe, **dadurch gekennzeichnet, dass** eine Vorrichtung nach einem der Ansprüche 1-16 verwendet wird.

24. Verfahren zur Extraktion von Nukleinsäure von einer in vitro amplifizierten Probe, **dadurch gekennzeichnet, dass** ein Kit nach einem der Ansprüche 17-18 verwendet wird.

25. Verfahren nach Anspruch 21 oder 22, **dadurch gekennzeichnet, dass** die Probe eine Probe aus Blut, Serum, Urin, Speichel, einer Zellsuspension und einer Biopsieprobe ist.

26. Verfahren nach Anspruch 21 oder 22, **dadurch gekennzeichnet, dass** die Probe menschliches Vollblut ist.

27. Verfahren zur Rekonstitution eines gefriergetrockneten Reagens, **dadurch gekennzeichnet, dass** eine Vorrichtung nach Anspruch 1 verwendet wird.

28. Verfahren zur Rekonstitution eines Reagens für eine der folgenden Reaktionen: Polymerase-Kettenreaktion (PCR), Ligase-Kettenreaktion (LCR), "unterbrochene-LCR-Reaktion", auf einer Nukleinsäuresequenz basierte Amplifikation (NASBA), sich selbst unterhaltende Replikation (SSR), Transkriptions-katalysierte Amplifikation (TMA), Strangverdrängungs-Amplifikation (SDA), Hybrid Capture^{®}-Raktion, Zielamplifikation, Signalamplifikation oder eine Kombination daraus, **dadurch gekennzeichnet, dass** eine Vorrichtung nach Anspruch 1 verwendet wird.

29. Verfahren für die Extraktion von Nukleinsäuren aus einer Probe, **dadurch gekennzeichnet, dass** die Probe in einem ersten Gefäß bereitgestellt wird, das abnehmbar mit einem zweiten Gefäß verbunden ist, wobei das zweite Gefäß ein im Voraus dispensiertes Reagens enthält, worauf die Probe in das zweite Gefäß und zurück gepumpt wird, wobei dies wiederholt wird, bis es ausreichend gemischt ist, wonach das Gefäß, das die Mischung enthält, an ein drittes Gefäß angebracht wird, das ein im Voraus dispensiertes Reagens enthält, und das Mischen wiederholt wird.

30. Verfahren nach Anspruch 29, **dadurch gekennzeichnet, dass** das zweite Gefäß einen Lysepuffer und eine Matrix enthält, dazu in der Lage, Nukleinsäure zu binden.

31. Verfahren nach Anspruch 29, **dadurch gekennzeichnet, dass** das dritte Gefäß einen Spülpuffer enthält.

32. Verfahren nach einem der Ansprüche 29-31, **dadurch gekennzeichnet, dass** die gereinigte Probe von einem Gefäß (3, 4) durch Zentrifugation entfernt wird.

33. Verfahren nach einem der Ansprüche 29-31, **dadurch gekennzeichnet, dass** die Matrix von der Flüssigkeit, in der sie suspendiert ist, durch Zentrifugation unter Verwendung einer Vorrichtung nach Anspruch 17 getrennt wird.

34. Verfahren nach einem der Ansprüche 29-31, **dadurch gekennzeichnet, dass** die Matrix von der Flüssigkeit, in der sie suspendiert ist, durch die Verwendung eines magnetischen Feldes getrennt wird.

35. Verfahren nach einem der Ansprüche 29-31, **dadurch gekennzeichnet, dass** die Probe eine der Folgenden ist: eine Probe aus Blut, Serum, Urin, Speichel, einer Zellsuspension, einer in vitro amplifizierten Probe oder einer Biopsieprobe.

36. Verfahren nach einem der Ansprüche 29-31, **dadurch gekennzeichnet, dass** die Probe eine Probe aus menschlichem Vollblut ist.

37. Verfahren zur Extraktion von spezifischen Nukleinsäurearten, **dadurch gekennzeichnet, dass** eine Vorrichtung nach Anspruch 1 verwendet wird, wobei die Vorrichtung eine Matrix enthält, die auf eine Art behandelt wird, die die Anheftung der Nukleinsäurearten an die Matrix begünstigt.

38. Verfahren zur Extraktion von spezifischen Nukleinsäuresequenzen, **dadurch gekennzeichnet, dass** eine Vorrichtung nach Anspruch 1 verwendet wird, wobei die Vorrichtung eine Matrix enthält, auf der eine komplementäre Nukleinsäuresequenz hybridisiert wird.

## Revendications

1. Appareil pour l'extraction d'acides nucléiques dans des échantillons organiques, **caractérisé en ce que** ledit appareil comprend un premier récipient (3) destiné à contenir l'échantillon, un premier piston (5) destiné à expulser ledit échantillon hors dudit premier récipient et au moins un second récipient (4), contenant au moins un réactif (2) alimenté au préalable, et ayant un second piston (6) destiné à expulser ledit réactif hors dudit second récipient, ledit premier récipient et ledit au moins un second récipient étant reliés de façon détachable par l'intermédiaire d'un passage étroit (7, 8), et ledit premier récipient et ledit au moins un second récipient définissant un volume fermé.

2. Appareil selon la revendication 1, **caractérisé en ce que** le réactif alimenté au préalable dans ledit second récipient est un réactif lyophilisé.

3. Appareil selon la revendication 1, **caractérisé en ce que** ledit second récipient contient en outre une matrice ayant des propriétés de liaison aux acides nucléiques.

4. Appareil selon la revendication 1, **caractérisé en ce que** le réactif alimenté au préalable dans ledit second récipient est un tampon de lyse et **en ce que** ledit second récipient contient en outre une matrice ayant des propriétés de liaison aux acides nucléiques.

5. Appareil selon l'une quelconque des revendications 3 ou 4, **caractérisé en ce que** ladite matrice est une matrice particulaire se déplaçant librement dans le récipient.

6. Appareil selon l'une quelconque des revendications 3 ou 4, **caractérisé en ce que** ladite matrice est constituée de particules ou de fibres primaires, formant une forme secondaire qui ne peut pas quitter le récipient.

7. Appareil selon la revendication 5, **caractérisé en ce que** ladite matrice est choisie parmi les particules de verre ou de silice, la diatomite, les fibres de verre, les fibres de nylon, une suspension de cellulose, des billes paramagnétiques et des billes de latex.

8. Appareil selon la revendication 3, **caractérisé en ce que** ladite matrice a reçu un traitement favorisant une affinité spécifique.

9. Appareil selon la revendication 3, **caractérisé en ce que** ladite matrice ne peut pas quitter le récipient en raison d'un champ magnétique agissant sur les composants magnétiques de la matrice.

10. Appareil selon la revendication 3, **caractérisé en ce que** la matrice est un bouchon de fibres de verre.

11. Appareil selon la revendication 8, **caractérisé en ce que** la matrice est revêtue de streptavidine.

12. Appareil selon la revendication 8, **caractérisé en ce que** la matrice porte des acides nucléiques hybridés.

13. Appareil selon la revendication 1, **caractérisé en ce que** le premier récipient (3) comprend des moyens de connexion (9, 11) destinés à enclencher des moyens correspondants (10, 12) sur le second récipient (4) ou sur d'autres récipients.

14. Appareil selon la revendication 13, **caractérisé en ce que** lesdits moyens de connexion (9, 11, 10, 12), en raison de leur taille et/ou de leur fonction, régulent la séquence de fixation du second récipient et des autres récipients.

15. Appareil selon la revendication 1, **caractérisé en ce que** les récipients (3, 4) et les autres récipients pouvant être fixés à ceux-ci possèdent des propriétés physiques telles que des marques tactiles, des codes en couleur, et analogues, guidant leur utilisation séquentielle.

16. Appareil selon la revendication 3, **caractérisé en ce qu'**il comprend un bouchon de séparation détachable (13) qui maintient la matrice solide.

17. Trousse comprenant un appareil selon la revendication 1 et un appareil (13) destiné à la séparation d'une matrice liant des acides nucléiques d'un liquide, ledit appareil ayant une extrémité ouverte destinée à une fixation à l'appareil selon la revendication 1, et une extrémité fermée munie d'au moins une ouverture de sortie, dans laquelle chacune desdites au moins une ouverture est située au-dessus de la surface intérieure la plus basse de l'appareil, définissant un volume qui n'est pas vidé par une centrifugation.

18. Trousse selon la revendication 17, **caractérisée en ce qu'**elle est fixée de façon sûre à l'extrémité (9 ou 10) de l' article (3 ou 4) selon la revendication 1 et s'adapte dans un tuyau de centrifugation.

19. Article selon l'une quelconque des revendications 1 - 16, **caractérisé en ce que** les récipients sont reliés pour former une grille correspondant au format standard de microtitration.

20. Trousse selon l'une quelconque des revendications 17 - 18, **caractérisée en ce que** les récipients sont reliés pour former une grille correspondant au format standard de microtitration.

21. Procédé d'extraction d'acides nucléiques d'un échantillon organique, **caractérisé en ce qu'**un article selon l'une quelconque des revendications 1 - 16 est utilisé.

22. Procédé d'extraction d'acides nucléiques d'un échantillon organique, **caractérisé en ce qu'**une trousse selon l'une quelconque des revendications 17 - 18 est utilisée.

23. Procédé d'extraction d'acides nucléiques d'un échantillon amplifié in vitro, **caractérisé en ce qu'**un article selon l'une quelconque des revendications 1 - 16 est utilisé.

24. Procédé d'extraction d'acides nucléiques d'un échantillon amplifié in vitro, **caractérisé en ce qu'**une trousse selon l'une quelconque des revendications 17 - 18 est utilisée.

25. Procédé selon la revendication 21 ou 22, **caractérisé en ce que** ledit échantillon est un échantillon de sang, de sérum, d'urine, de salive, une suspension de cellules et un échantillon de biopsie.

26. Procédé selon la revendication 21 ou 22, **caractérisé en ce que** ledit échantillon est du sang humain complet.

27. Procédé de reconstitution d'un réactif lyophilisé, **caractérisé en ce qu'**un article selon la revendication 1 est utilisé.

28. Procédé de reconstitution d'un réactif pour l'une quelconque des réactions suivantes : une réaction en chaîne de polymérase (PCR), une réaction en chaîne de ligase (LCR), "une réaction LCR partagée", une amplification à base d'une séquence d'acides nucléiques (NASBA), un e duplication auto-retardée (SSR) , une amplification réalisée par l'intermédiaire d'une transcription (TMA), une amplification par déplacement de brins (SDA), une réaction de Capture® hybride, une amplification de cible, une amplification de signal ou une combinaison de celles-ci, **caractérisé en ce qu'**un article selon la revendication 1 est utilisé.

29. Procédé d'extraction d'acides nucléiques d'un échantillon, **caractérisé en ce que** ledit échantillon est chargé dans un premier récipient, qui est relié de façon détachable à un second récipient, ledit second récipient contenant un réactif alimenté au préalable, à la suite de quoi l'échantillon est pompé dans le second récipient et ensuite renvoyé, ce cycle étant répété jusqu'à ce qu'il soit suffisamment mélangé, à la suite de quoi le récipient contenant le mélange est fixé à un troisième récipient contenant un réactif alimenté au préalable et le mélange est répété.

30. Procédé selon la revendication 29, **caractérisé en ce que** le second récipient contient un tampon de lyse et une matrice, capable de lier des acides nucléiques.

31. Procédé selon la revendication 29, **caractérisé en ce que** le troisième récipient contient un tampon de rinçage.

32. Procédé selon l'une quelconque des revendications 29 - 31, **caractérisé en ce que** l'échantillon purifié est retiré d'un récipient (3, 4) par centrifugation.

33. Procédé selon l'une quelconque des revendications 29 - 31, **caractérisé en ce que** la matrice est séparée du liquide, dans lequel elle est mise en suspension, par centrifugation à l' aide d'un article selon la revendication 17.

34. Procédé selon l'une quelconque des revendications 29 - 31, **caractérisé en ce que** la matrice est séparée du liquide, dans lequel elle est mise en suspension, à l'aide d'un champ magnétique.

35. Procédé selon l'une quelconque des revendications 29 - 31 , **caractérisé en ce que** l'échantillon est l'un des échantillons suivants : un échantillon de sang, du sérum, de l'urine, de la salive, une suspension de cellules, un échantillon amplifié in vitro ou un échantillon de biopsie.

36. Procédé selon l'une quelconque des revendications 29 - 31 , **caractérisé en ce que** l'échantillon est un échantillon de sang humain complet.

37. Procédé d'extraction d'acides nucléiques spécifiques, **caractérisé en ce qu'**un article selon la revendication 1 est utilisé, ledit article contenant une matrice traitée d'une manière favorisant la fixation desdits acides nucléiques à ladite matrice.

38. Procédé d'extraction de séquences d'acides nucléiques spécifiques, **caractérisé en ce qu'**un article selon la revendication 1 est utilisé, ledit article contenant une matrice portant une séquence d'acides nucléiques complémentaire hybridée sur celle-ci.
